# EUROPEAN PATENT APPLICATION

(11) **EP 3 338 816 A1**
(43) Date of publication of application: **27.06.2018**
(21) Application number: 17199767.9
(22) Date of filing: 09.09.2010
(51) Int. Cl.: A61L 27/16, A61L 27/54, A61C 8/00

(54) **COMPOSITIONS AND METHODS FOR DETECTING AND TREATING IMPLANT LOOSENING AND OSTEOLYSIS**

(30) Priority: 09.09.2009 US 276213 P
(62) Divisional of application: 10816063.1
(71) Applicant: Board Of Regents Of The University Of Nebraska, Omaha, NE 68198-6099 (US); Hospital for Special Surgery, New York, NY 10021 (US)
(72) Inventor: Wang, Dong, Omaha, Nebraska 68154 (US); Goldring, Steven R, New York, NY New York 10021 (US); Fehringer, Edward V, Omaha, NE Nebraska 68144 (US)
(74) Representative: Potter Clarkson LLP

(57) **Abstract**

A method of detecting implant loosening in a subject, or determining an increased risk for osteolysis in a subject, said method comprising administering to the subject a composition comprising at least one water soluble polymer and at least one pharmaceutically acceptable carrier, wherein said water soluble polymer is operably linked to at least one imaging agent.

A method of inhibiting the loosening of an implant in a subject, or inhibiting osteolysis in a subject, said method comprising administering to the subject a composition comprising at least one water soluble polymer and at least one pharmaceutically acceptable carrier, wherein said water soluble polymer is operably linked to at least one therapeutic agent.

## Description

This application claims priority under 35 U.S.C. §119(e) to U.S. Provisional Patent Application No. 61/276,213, filed on September 9, 2009. The foregoing application is incorporated by reference herein.

This invention was made with government support under Grant No. R01 AR053325 awarded by the National Institutes of Health. The government has certain rights in the invention.

### FIELD OF THE INVENTION

The present invention relates to the fields of osteolysis and implant loosening. More specifically, the invention provides compositions and methods for detecting and treating implant loosening and osteolysis.

### BACKGROUND OF THE INVENTION

Several publications and patent documents are cited throughout the specification in order to describe the state of the art to which this invention pertains. Each of these citations is incorporated herein by reference as though set forth in full.

It is estimated that 1.5 million total joint arthroplasty procedures are performed annually worldwide to treat end-stage joint disease (Drees et al. (2007) Nat. Clin. Pract. Rheumatol., 3:165-71). The overall 10-year success rate for total joint replacement is 90% with close to 10% of patients requiring revision surgery, which is associated with a poorer outcome and a shorter duration of implant survival (Santerre et al. (2000) Can. J. Surg., 43:173-9). Inflammation caused by wear particles generated from the articulating surfaces of prosthetic components is considered to represent the major cause of aseptic implant loosening and clinical failure after total joint replacement (Holt et al. (2007) Clin. Orthop. Relat. Res., 460:240-52). Wear particles have been shown to activate macrophages and induce a granulomatous inflammatory reaction that results in osteoclast-mediated peri-implant osteolysis at the bone-implant interface, leading to the loss of the fixation.

Non-invasive imaging modalities such as successive x-ray and CT have been used in the clinical diagnosis of osteolysis and implant loosening (Leopold et al. (1999) Clin. Orthopaed. Rel. Res., 179-86). These methods are effective in detecting osteolysis and associated loss of implant fixation. At early stages of osteolysis, however, the skeletal changes are difficult to detect and imaging procedures are costly and accompanied by high radiation exposure, and there is a need for more sensitive techniques to detect early particle-induced inflammation prior to the development of extensive osteolysis.

### SUMMARY OF THE INVENTION

In accordance with the present invention, methods of detecting implant loosening are provided. In a particular embodiment, the method comprises administering to a subject a composition comprising at least one water-soluble polymer and at least one pharmaceutically acceptable carrier, wherein the water-soluble polymer is operably linked to at least one imaging agent and wherein the presence of the water-soluble polymer at the site of the implant is indicative of implant loosening. In a particular embodiment, the water-soluble polymer is a N-(2-hydroxypropyl)methacrylamide (HPMA) copolymer.

In accordance with another aspect of the instant invention, methods of determining an increased risk for osteolysis are provided (e.g., providing a diagnosis or prognosis). In a particular embodiment, the method comprises administering to a subject a composition comprising at least one water soluble polymer and at least one pharmaceutically acceptable carrier, wherein the water soluble polymer is operably linked to at least one imaging agent and wherein the localization of the water soluble polymer at a site in the subject is indicative of an increased risk for osteolysis. In a particular embodiment, the subject has an orthopedic (e.g., joint replacement) and/or dental implant. In a particular embodiment, the water soluble polymer is a *N*-(2-hydroxypropyl)methacrylamide (HPMA) copolymer.

In yet another aspect, methods of inhibiting the loosening of an implant are provided. In a particular embodiment, the method comprises administering to a subject a composition comprising at least one water soluble polymer and at least one pharmaceutically acceptable carrier, wherein the water soluble polymer is operably linked to at least one therapeutic agent. In a particular embodiment, the therapeutic agent is an anti-inflammatory therapeutic agent such as dexamethasone. The water soluble polymer may be operably linked to the anti-inflammatory therapeutic agent via a degradable/cleavable linker such as a pH-sensitive linker. In a particular embodiment, the water soluble polymer is a *N*-(2-hydroxypropyl)methacrylamide (HPMA) copolymer. Additionally, the methods may further comprise administering of at least one additional anti-inflammatory therapeutic agent.

In accordance with another aspect of the present invention, methods of inhibiting osteolysis in a subject are provided. In a particular embodiment, the method comprises administering to the subject a composition comprising at least one water soluble polymer and at least one pharmaceutically acceptable carrier, wherein said water soluble polymer is operably linked to at least one therapeutic agent. In a particular embodiment, the therapeutic agent is an anti-inflammatory therapeutic agent such as dexamethasone. The water soluble polymer may be operably linked to the anti-inflammatory therapeutic agent via a degradable/cleavable linker such as a pH-sensitive linker. In a particular embodiment, the water soluble polymer is a *N*-(2-hydroxypropyl)methacrylamide (HPMA) copolymer. Additionally, the methods may further comprise administering of at least one additional anti-inflammatory therapeutic agent. The osteolysis may be at the site of an orthopedic or dental implant.

### BRIEF DESCRIPTION OF THE DRAWINGS

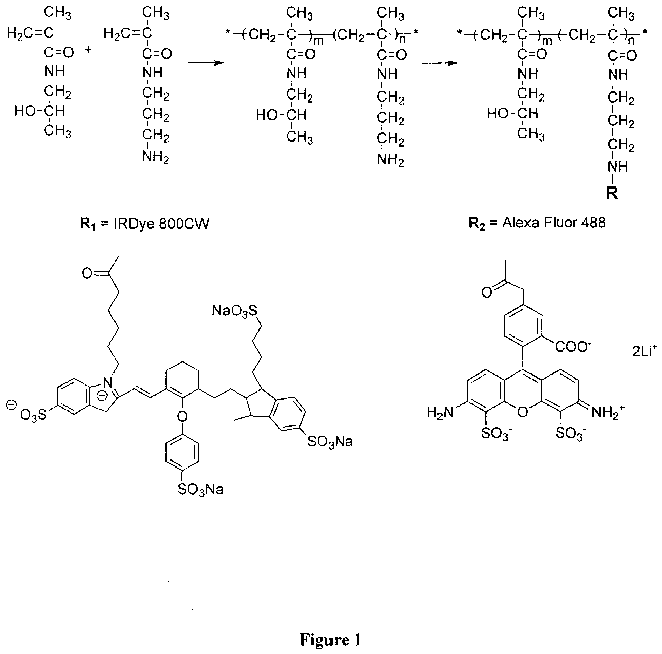

Figure 1 is a schematic of the syntheses of P-IRDye and P-Alexa.

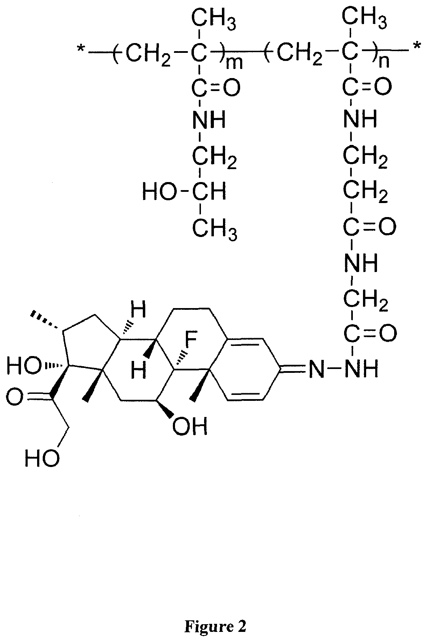

Figure 2 provides the chemical structure of P-Dex.

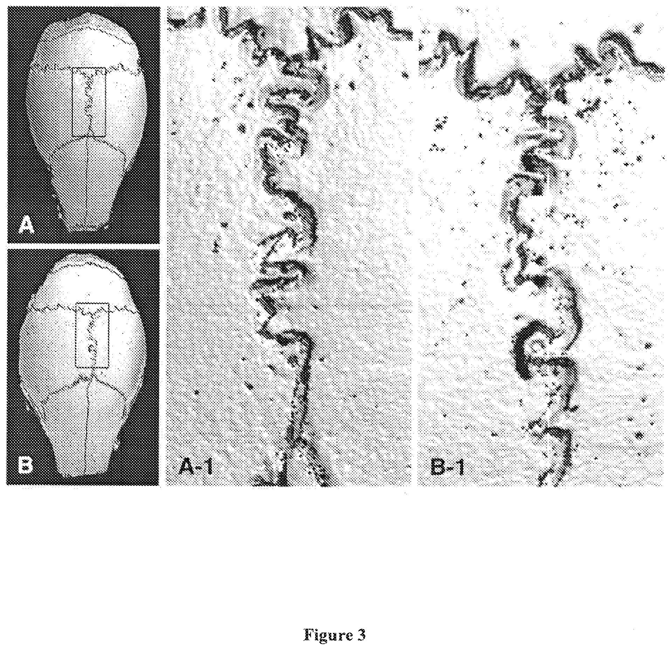

Figure 3 provides representative micro-computer tomography (micro-CT) images of the mouse calvaria 7 days after implantations with PBS (Figs. 3A and 3A-1) or PMMA particle (Figs. 3B and 3B-1). Figures 3A-1 and 3B-1 are enlargements of the selected regions in Figures 3A and 3B, respectively. The PMMA particle-implanted animals showed strong evidence of bone resorption compared to the PBS-treated animals. In addition, there were significant differences between the two groups in terms of bone surface to bone volume (BS/BV) and bone thickness (p < 0.05).

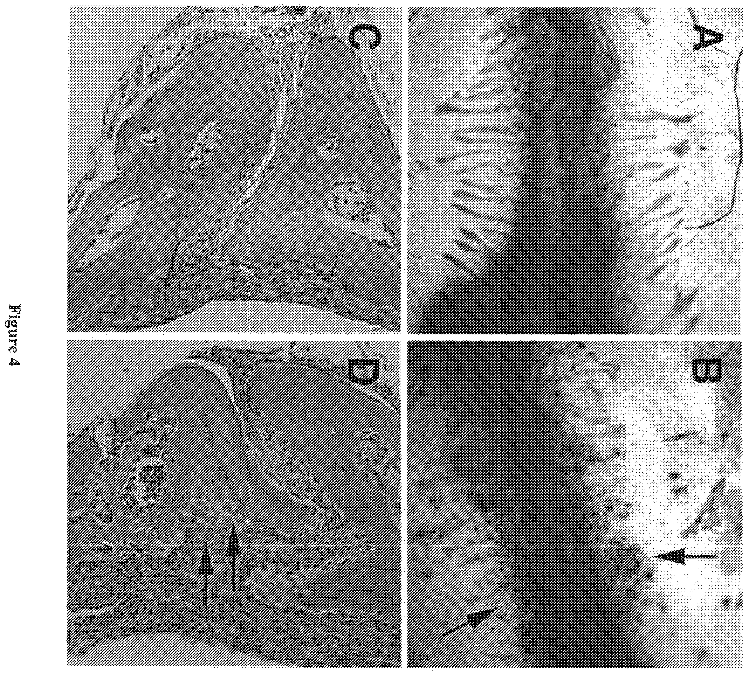

Figure 4 provides representative images of undecalcified calvaria after tartrate-resistant acid phosphatase (TRAP)-staining (Figs. 4A and 4B) and hematoxylin and eosin (H&E) stained decalcified calvaria tissue sections (Figs. 4C and 4D). TRAP-staining of the undecalcified calvarial tissue shows the presence of abundant TRAP-positive tissue (Fig. 4B) demonstrated by the arrow (magnifications are at 40x). After decalcification, calvaria sections were H&E stained (Figs. 4C and 4D). The double arrow in Figure 4D indicates the region of focal bone resorption (400x).

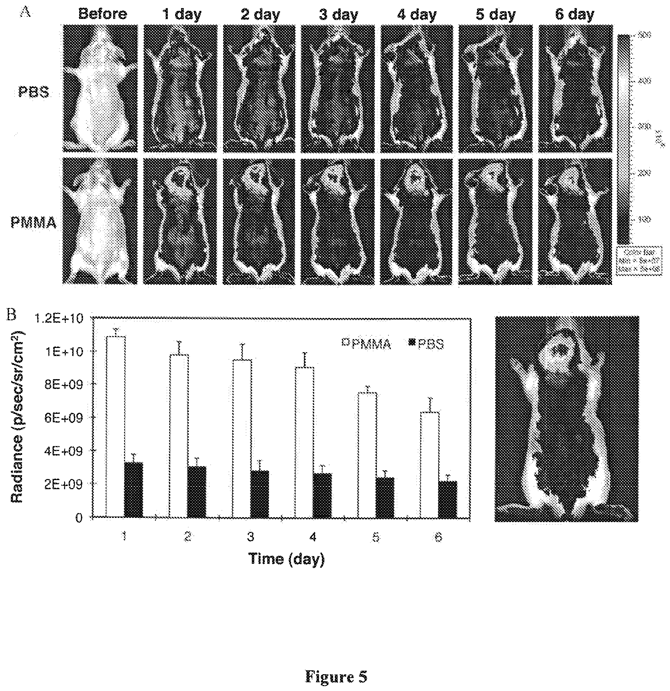

Figure 5 provides live optical imaging after phosphate-buffered saline (PBS) or poly(methyl methacrylate) (PMMA) implantation. P-IRDye was given via tail vein injection the following day after PBS or PMMA implantation. The mice were imaged prior and each day after the administration of the optical imaging agent for the following 6 days. In Figure 5A, the upper panel shows the images from the PBS-treated group. The lower panel shows the images from the PMMA-particle implanted group. Compared to the PBS group, PMMA particle implanted animals demonstrated more intense and longer lasting NIR signals in the calvarial region where the PMMA particles were implanted. Figure 5B shows the NIR signal intensity was measured from a consistent region of interest (circle) in the calvaria site for all the mice. The signal intensity differences in the two groups were statistically significant (p < 0.05).

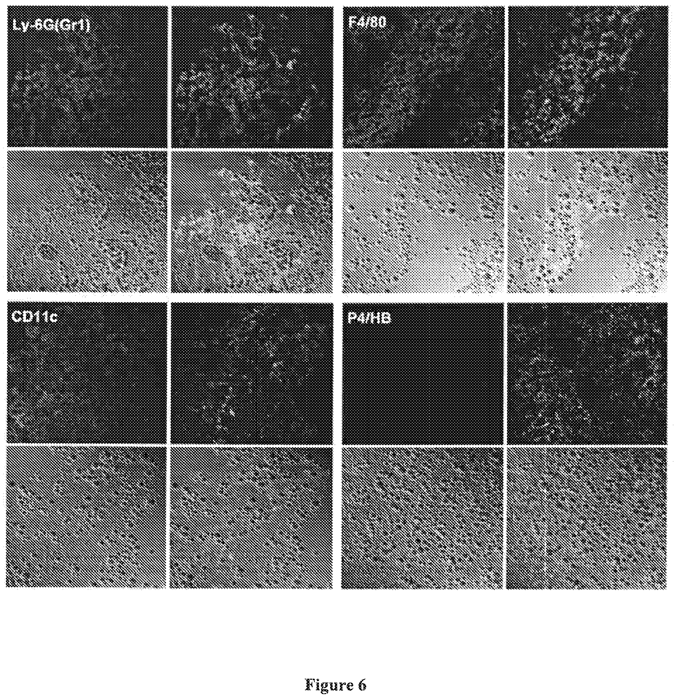

Figure 6 provides representative confocal images of anti-Ly-6G (Gr-1, Gr1), anti-F4/80, anti-CD 11c and anti-P4HB antibody stained frozen sections of calvaria and adjacent soft tissue from PMMA particle implanted mice (treated with P-Alexa). Each panel was composed of four sub-images: antibody red staining, P-Alexa green fluorescence, DIC image and the co-localization of the three. The colocalization of red and green color in both panels yields a yellow color, which confirms the internalization of the HPMA copolymer conjugate by Ly-6G (Gr-1, Gr1), F4/80 or CD11c positive cells at the sites of inflammation. Magnifications are at 400x.

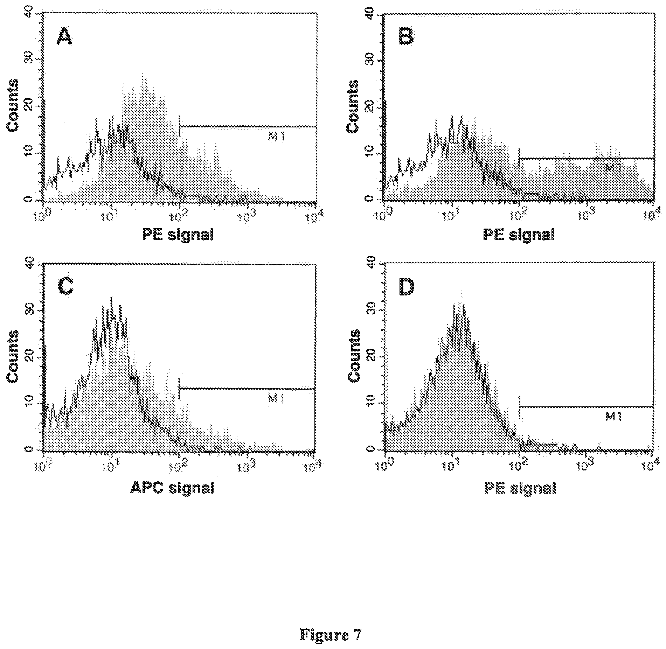

Figure 7 provides representative data from fluorescence-activated cell scanning (FACS) analysis of cells isolated from sites of PMMA particle-induced inflammation 24 hours after systemic administration of P-Alexa. The histogram plots show the intensity of staining with the specific antibodies designated on the x-axis (fill) with isotype control antibodies (lines) on the same plots. The percentages represent the percent of antibody positive cells among P-Alexa positive cells. Figure 7A: 25.12% P-Alexa positive cells were F4/80 positive; Figure 7B: 35.15 % P-Alexa positive cells were Ly-6G positive; Figure 7C: 9.73% P-Alexa positive cells were CD11c positive; Figure 7D: < 1% P-Alexa positive cells were P4HB positive.

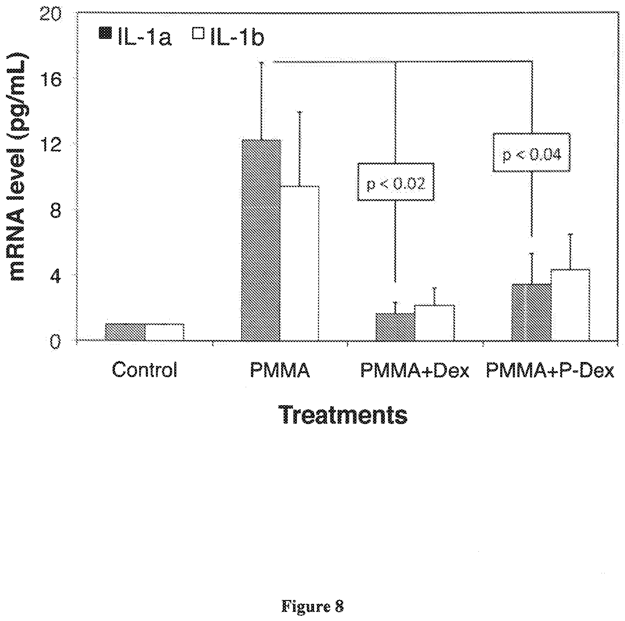

Figure 8 demonstrates the effects of P-Dex on suppression of PMMA particle-induced IL-1α and IL-1β mRNA in cultured human monocytes. mRNA expression levels are expressed relative to the housekeeping gene GAPDH, and normalized to untreated control cells. n = 3. The IL-1α mRNA level of PMMA group is significantly higher than the free Dex and the P-Dex groups (p < 0.05). No significant difference was detected between free Dex and P-Dex groups. The IL-1β mRNA level of PMMA group is also higher (but not significantly) than the free Dex and the P-Dex groups.

### DETAILED DESCRIPTION OF THE INVENTION

Herein, it is demonstrated that the N-(2-hydroxypropyl)methacrylamide (HPMA) copolymer localize to sites of granulomatous inflammation, such as that induced by wear particles. Recent data have demonstrated that the HPMA copolymer (Duncan, R. (2003) Nature Rev., 2:347-60) selectively accumulates at inflammatory sites in an adjuvant-induced arthritis (AA) rat model and when conjugated to a contrast agent the copolymer can effectively image sites of articular inflammation (Wang et al. (2007) Arthritis Res. Ther., 9:R2; Liu et al. (2008) Pharm. Res., 25:2910-9). When loaded with dexamethasone (Dex, a potent anti-inflammatory glucocorticoid), the HPMA copolymer conjugate (P-Dex) provides superior and sustained amelioration of the inflammation when compared to an equivalent dose of free Dex. Indeed, the HPMA copolymer system was used in a mouse model of particle-induced osteolysis and it is shown that the HPMA copolymer system can be used effectively for detection of local particle-induced inflammation and for targeting a therapeutic anti-inflammatory agent to the site of inflammation to prevent osteolysis. HPMA copolymers tagged with imaging probes may be used for early detection of peri-prosthetic osteolysis. The system may be adapted to use gamma or positron emitters as reporting mechanism in mammalian (e.g., human) subjects to detect early wear-particle-induced peri-implant granuloma formation and early signs of osteolysis. For later stages of osteolysis which may not be associated with severe inflammation, polymeric carriers with bone-targeting moieties can be used to target the site of early bone lesion.

As stated hereinabove, wear particle-induced inflammation is considered to be the major cause of aseptic implant loosening and clinical failure after total joint replacement. Due to the frequent absence of symptoms, early detection and intervention prior to implant failure presents a significant challenge. To address this issue, a HPMA copolymer-based optical imaging contrast agent (e.g., P-IRDye) was developed and used for the detection of wear particle-induced inflammation. Biocompatible water-soluble polymers, such as HPMA copolymer, can specifically localize to sites of joint inflammation, using an adjuvant-induced arthritis (AA) rat model (Wang et al. (2004) Pharm. Res., 21:1741-9). Wear particle-induced inflammation was studied employing a murine osteolysis model in which poly(methyl methacrylate) (PMMA) particles are implanted onto the calvaria of Swiss Webster mice. More specifically, a standard mouse calvaria osteolysis model, which has been widely used for the study of aseptic implant loosening, was modified (Kaar et al. (2001) J. Orthop. Res., 19:171-8; von Knoch et al. (2005) Biomaterials 26:5783-9). In this model, a midline sagittal incision is made to allow calvaria exposure, followed by periosteum removal, particle deposition and incision closure. Two factors contribute to the inflammation at the implantation site, the natural repair of the surgical trauma and the cellular and tissue reaction to the implanted particles. To minimize the effects of the surgical trauma, this procedure was modified and a minimally invasive method was used to introduce PMMA particles onto the mouse calvaria. This modified procedure includes two steps: removal of periosteum by the inserted needle tip and instillation of the PMMA particles via the needle. The particle-induced osteolysis was confirmed by micro computer tomography (µ-CT), hematoxylin and eosin (H&E) and tartrate-resistant acid phosphatase (TRAP) staining of calvaria isolated at necropsy. After PMMA particle implantation, P-IRDye was administrated to the mice via tail vein injection. Live imaging of the animals after implantation revealed the preferential distribution and sustained retention of the macromolecular contrast agent at the site of particle implantation. Immunohistochemical staining and FACS analyses of the calvaria-associated soft tissue revealed extensive uptake of the HPMA copolymer by F4/80, Ly-6G (Grl) and CD11c positive cells, which accounts for the sustained retention of the macromolecular probes at the inflammatory sites. To test the potential of the system for therapeutic intervention, an acid-labile HPMA copolymer-dexamethasone conjugate (P-Dex) was prepared and shown to prevent PMMA-induced inflammation and osteolysis in the calvarial implant model. An *in vitro* cell culture model was used to demonstrate that P-Dex pretreatment suppressed IL-1α and IL-1β expression in PMMA challenged monocytes, which provides a potential explanation for the *in vivo* efficacy of P-Dex in inhibiting PMMA-induced osteolysis.

The *in vivo* optical imaging studies with the modified mouse calvaria osteolysis model revealed that P-IRDye was mainly localized to the PMMA particle implantation sites 7 days post particle implantation. By this time, soft tissue was inflamed with clearly visual evidence of swelling and redness. H&E stained tissue sections from the particle implantation sites revealed infiltration of abundant inflammatory cells associated with the local tissue inflammation. Daily imaging of the animals revealed that the P-IRDye signal at the particle implantation site was sustained during the entire course of the study, with a gradual decline to 60% of the original signal intensity by day 6 post P-IRDye administration. The sustained contrast signal level indicates the clinical utility of this imaging system since it provides an extended period of time for detection. While this polymer-based imaging approach may be used for early diagnosis of implant associated inflammation, the selection of the near infrared imaging probe is preferably adapted for alternate imaging agents for clinical application in human subjects since near infrared imaging has lower tissue penetration capability, especially through mineralized tissues, than other imaging agents (Kolari et al. (1993) Acupunct. Electrother. Res., 18:17-21; Mancini et al. (1994) J. Appl. Physiol., 77:2740-7). As an alternative, gamma or positron emitters (e.g. ¹²³I, ¹¹¹In, ^{99m}Tc, ¹⁸F, ⁶⁴Cu, ²⁰¹Tl, etc.) may be utilized in mammalian, large animal, or human clinical applications. Due to the high energy levels of these radioisotopes, their signals can be readily detected by clinical imaging modalities such as single photon emission CT/CT (SPECT/CT) and positron emission tomography - computed tomography (PET/CT). Magnetic resonance imaging (MRI) may also be used to detect the polymers of the instant invention.

Similar to the enhanced permeability and retention (EPR) effect observed with solid tumors (Matsumura et al. (1986) Cancer Res., 46:6387-92), increased vascular permeability to macromolecules has been well-documented with inflammatory tissues (Henson, P.M. (2005) Nat. Immunol., 6:1179-81). Different from solid tumors, however, the clearance of macromolecules from the interstitial space of inflammatory tissues proceeds rapidly and steadily via the lymphatic system, after extravasation from blood vessels (Maeda, H. (2010) Bioconjugate Chem., 21:797-802). The finding of the P-IRDye's sustained signal at the inflammatory sites in this study is of great significance since it indicates the presence of a previously unrecognized mechanism of macromolecule and other colloidal systems retention at sites of inflammation. To explore this novel mechanism, mouse calvaria tissues associated with PMMA particles were isolated and processed for histological and FACS analyses. P-Alexa was used as a surrogate for P-IRDye as the near infrared signal could not be detected by a confocal microscope and flow cytometer. To ensure similar physical-chemical properties of the two conjugates, the same amine-containing HPMA copolymer precursor used in the synthesis of P-IRDye was also used in the synthesis of P-Alexa. Immunohistological evaluation revealed that P-Alexa was internalized by cells at the inflammatory sites. Using a panel of antibodies, the cells were identified as macrophages (F4/80⁺), dendritic cells (CD11c⁺) or inflammatory monocytes (Gr1⁺). Although Gr1 is also expressed on neutrophils, H&E staining (e.g. Figure 4D) confirmed that cells expressing phenotypic features of neutrophils were rarely detected. Meanwhile, FACS analysis using specific neutrophil marker (rat anti-mouse neutrophils marker 7/4, AbD Serotec, Raleigh, NC) also showed less than 5% percent of the P-Alexa positive cells were 7/4 positive. Therefore, the GR1 and P-Alexa-positive cells were predominantly inflammatory monocytes. Analysis of the H&E tissue sections demonstrated that there were cells with phenotypic features of fibroblasts, though P4HB positive cells were not detected in the immunohistological and FACS analyses, which may be attributed to the antigen denaturation during the tissue/cell processing. FACS analysis of cells dispersed from the sites of particle implantation showed that majority of the cells (more than 80%) were positive for Ly-6G (Gr-1, Gr1), which is consistent with the acute inflammatory response in the immediate period after the PMMA particle implantation. These findings indicate that the retention of the polymeric imaging probes is related to the cellular uptake of the copolymer by activated inflammatory cells after extravasation at the inflammatory site.

Additional studies were undertaken to determine whether this novel retention mechanism associated with the particle-induced inflammation could be exploited for development of a therapeutic intervention to inhibit particle-induced inflammation and associated osteolysis. The micro-CT results demonstrated that administration of a single P-Dex one day after particle implantation attenuated osteolysis. *In vitro* cell culture studies showed that P-Dex was internalized by PMMA-treated human monocytes via endocytosis and that it was retained in a lysosomal compartment. This treatment was accompanied by the suppression of particle-induced expression of inflammatory cytokines such as IL-1α and IL-1β, which likely contribute to the attenuation of the local inflammatory response. Suppression of expression of IL-1 and related inflammatory cytokines with osteoclast-inducing activity by the P-Dex, provides a likely mechanism by which the P-Dex copolymer inhibited particle-induced osteolysis.

Using a modified murine calvarial osteolysis model, it has been found herein that imaging agents based on water-soluble macromolecules, such as HPMA copolymers, can be used to identify sites of inflammation associated with the early stage of particle-induced inflammation and subsequent osteolysis. A novel inflammatory cell-mediated macromolecule retention mechanism associated with this recognition was identified. A HPMA copolymer based dexamethasone prodrug was shown to provide sustained suppression of the inflammation associated with implanted PMMA particles. Micro-CT analysis revealed that systemic administration of the P-Dex to the murine calvarial osteolysis model was osteo-protective. Adaptation of this system for the use of high-energy radioisotopes instead of optical imaging probe superior imaging tools for human application. Additionally, the instant studies demonstrate the ability of this macromolecular theranostic system for the tissue specific delivery of biologically active drugs to sites of inflammation to prevent bone destruction.

While HPMA copolymers (e.g., HPMA-APMA copolymers; see also Figures 1 and 2) are exemplified throughout the instant application, other water-soluble polymer backbones or colloidal systems may be used (e.g., linked to the imaging, therapeutic, and/or targeting agents described herein). Water-soluble polymers of the instant invention include, but are not limited to, polymers comprising a methyl acrylamide backbone, a HPMA copolymers and derivatives, polyethylene glycol (including branched or block copolymers, which may be degradable via peptide sequences, ester or disulfide bonds, etc.), polyglutamic acid, polyaspartic acid, dextran, chitosan, cellulose and its derivatives, starch, gelatin, hyaluronic acid and its derivatives, and polymers or copolymers of the following monomers: N-isopropylacrylamide (e.g., PNIPAm), acrylamide, N,N-dimethylacrylamide, N-vinylpyrrolidone (e.g., PVP), vinyl acetate (e.g., resulting polymer hydrolyzed into polyvinyl alcohol or PVA), hydroxyethylmethacrylate (e.g., PHEMA), 2-methacryloxyethyl glucoside, acrylic acid, methacrylic, vinyl phosphonic acid, styrene sulfonic acid, maleic acid, 2-methacrylloxyethyltrimethylammonium chloride, methacrylamidopropyltrimethyl-ammonium chloride, methacryloylcholine methyl sulfate, N-methylolacrylamide, 2-hydroxy-3-methacryloxypropyltrimethyl ammonium chloride, 2-methacryloxyethyl-trimethylammonium bromide, 2-vinyl-1-methylpyridinium bromide, 4-vinyl-1-methyl-pyridinium bromide, ethyleneimine, (N-acetyl)ethyleneimine, (N-hydroxyethyl) ethyleneimine and/or allylamine. Preferably, the water-soluble polymer is biologically inert, however, optionally the polymer may have therapeutic activity (Rapp et al., Synthesis and in vivo biodisposition of [14C]-quatemary ammonium-melphalan conjugate, a potential cartilage-targeted alkylating drug, Bioconjug Chem. (2003) 14(2):500-6). Colloidal systems/carriers include, without limitation, liposomes, nanoparticles, and micelles (optionally cross-linked).

In a particular embodiment of the instant invention, the polymers of the instant invention are copolymers comprising a methacrylamide backbone, wherein the methacrylamide units have alkyl or aryl side chains. In a particular embodiment, the amide group of the methacrylamide backbone is omitted. The polymers may comprise at least one therapeutic agent, at least one imaging agent, and/or at least one targeting moiety, all optionally linked via an independently selected spacer/linker. In one embodiment of the instant invention, the polymers of the complexes are block copolymers. Block copolymers are most simply defined as conjugates of at least two different polymer segments (Tirrel, M. In: Interactions of Surfactants with Polymers and Proteins. Goddard E.D. and Ananthapadmanabhan, K.P. (eds.), CRC Press, Boca Raton, Ann Arbor, London, Tokyo, pp. 59-122, 1992). The simplest block copolymer architecture contains two segments joined at their termini to give an A-B or B-A type diblock. Consequent conjugation of more than two segments by their termini yields A-B-A type triblock, A-B-A-B-type multiblock, multisegment A-B-C architectures, and the like. More complex architectures such as (AB)ₙ or AₙBₘ starblocks which have more than two polymer segments linked to a single center, are also encompassed by the instant invention.

In another embodiment, the polymer of the instant invention comprises the general structure: 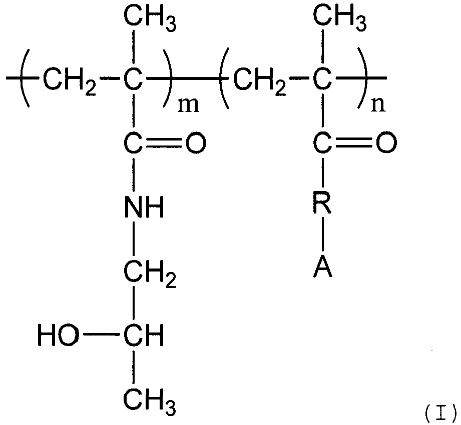 wherein R is a linker; A is an imaging agent, targeting moiety, or a therapeutic agent; and m and n are independently from about 1 to about 1000, preferably about 10 to about 500. In a particular embodiment, R is an alkyl, aryl, or polypeptide. In a particular embodiment, the R group comprises a pH sensitive linker and/or a cleavable linker. A single copolymer of the instant invention may comprise multiple blocks of formula (I) linked together (e.g., about 2 to about 1000, about 2 to about 500, about 2 to about 10, about 2 to about 5). A single copolymer of the instant invention may comprise at least one imaging agent, at least one targeting moiety, and/or at least one therapeutic agent. For example, a single polymer may comprise one HPMA block, a block comprising an imaging agent, and a block comprising a targeting moiety (e.g., the copolymer would have two "n" blocks - an A-B-B' block copolymer). Additionally, while formula (I) depicts the HPMA block first (A-B), other blocks may precede the HPMA block (e.g., B-A, B-A-B, etc.).

The polymers of the instant invention may, optionally, include one or more targeting moieties, which may be used to direct the delivery system to a specific tissue, such as bone, tooth, cartilage, or certain cell types, etc. Preferably, targeting moieties are those compounds which preferentially accumulate in/on hard tissue (e.g., tooth and bone) and/or medical implants (e.g., bone graft/implant, hydroxyapatite-coated metal implant, metal implants such as stainless steel, titanium alloy, orthopedic implants, dental implants, and bone marrow grafts) rather than any other organ or tissue *in vivo.* Targeting moieties of the instant invention include, without limitation, folic acid, mannose, bisphosphonates (e.g., alendronate), quaternary ammonium groups, tetracycline and its analogs, sialic acid, malonic acid, N,N-dicarboxymethylamine, 4-aminosalicyclic acid, 5-aminosalicyclic acid, antibodies or fragments or derivatives thereof specific for hard tissue or implant material (e.g., Fab, humanized antibodies, and/or single chain variable fragment (scFv)), and peptides (e.g., peptides comprising about 2 to about 100 (particularly 6) D-glutamic acid residues, L-glutamic acid residues, D-aspartic acid residues, L-aspartic acid residues, D- phosphoserine residues, L-phosphoserine residues, D- phosphothreonine residues, L-phosphothreonine residues, D-phosphotyrosine residues, and/or L- phosphotyrosine residues). In a particular embodiment, the targeting moiety is alendronate. A targeting moiety may be linked to the polymer backbone via covalent or physical bonds (linkages). Optionally, the spacers between a targeting moiety and the polymer backbone may be cleaved upon a stimulus including, but not limited to, changes in pH (e.g., an acid-labile linker), presence of a specific enzyme activity (for example, cathepsins (e.g., cathepsin K), MMPs, etc.), changes in oxygen levels, the presence of light of certain wavelength, etc.

As stated hereinabove, the HPMA copolymers of the instant invention can be used for the delivery of at least one therapeutic agent (drug) to the diseased sites for the treatment of osteolysis, the inflammation associated therewith, and/or implant loosening. In another embodiment, the HPMA copolymers can be used for delivery of at least one imaging agent to a desired site for non-invasive imaging and early detection of or assessing the risk of osteolysis and implant loosening. The HPMA copolymers of the instant invention may each comprise at least one therapeutic agent and/or imaging agent. In another embodiment, multiple HPMA copolymers are administered (simultaneously or sequentially) each of which comprises a single therapeutic agent and/or imaging agent.

In a particular embodiment, detection agents/imaging agents (or labels) may be attached to the HPMA copolymer backbone. In a particular embodiment, the average mol percentage per polymer chain may range from 0% to about 50%. The imaging agents may be compounds useful for optical imaging, magnetic resonance imaging (MRI), positron emission tomography (PET), computerized tomography (CT), gamma-scintigraphy imaging, and the like. Such agents are well-known to those of skill in the art. Imaging agents include, without limitation, radioisotope, isotopes, biotin and derivatives thereof, gold (e.g., nanoparticles), optical imaging agents (e.g., near IR dyes (e.g., IRDye 800CW) phorphyrins, anthraquinones, anthrapyrazoles, perylenequinones, xanthenes, cyanines, acridines, phenoxazines, phenothiazines and derivatives thereof), chromophore, fluorescent compounds (e.g., Alexa Fluor® 488, fluorescein, rhodamine, DiI, DiO, and derivatives thereif), MRI enhancing agents (for example, DOTA-Gd3⁺ (1,4,7,10-tetraazacyclododecane-1,4,7,10-tetra (acetic acid)), DTPA-Gd3⁺ (gadolinium complex with diethylenetriamine pentaacetic acid), etc.), paramagnetic or superparamagnetic ions (e.g., Gd(III), Eu(III), Dy(III), Pr(III), Pa(IV), Mn(II), Cr(III), Co(III), Fe(III), Cu(II), Ni(II), Ti(III), and V(IV)), PET compounds labeled or complexed with ¹¹C, ¹³N, ¹⁵O, ¹⁸F, ⁶⁴Cu, ⁶⁸Ga, or ⁸²Rb (e.g., ¹⁸F-FDG (fluorodeoxyglucose)), CT compounds (for example, iodine or barium containing compounds, e.g., 2,3,5-triiodobenzoic acid), and gamma or positron emitters (for example, ^{99m}Tc, ¹¹¹In, ¹¹³In, ¹⁵³Sm, ¹²³I, ¹³¹I ¹⁸F, ⁶⁴Cu, ²⁰¹Tl, etc.).

In a particular embodiment, the therapeutic agent attached to the HPMA copolymer of the instant invention is an anti-inflammatory therapeutic agent, immunosuppressant, pain management drug, an anabolic factor (e.g., growth factors that promote tissue regeneration and repair, such as bone morphogenetic proteins (BMPs), Wnt pathway agonists, insulin-like growth factor 1 (IGF-1), fibroblast growth factors (FGFs), platelet-derived growth factor (PDGF), and agents targeting bone resorption (e.g., bisphosphonate)), or a bone related therapeutic agent. As used herein, an "anti-inflammatory therapeutic agent" refers to compounds for the treatment of an inflammatory disease or the symptoms associated therewith. Anti-inflammatory therapeutic agents include, without limitation, non-steroidal anti-inflammatory drugs (NSAIDs; e.g., aspirin, ibuprofen, naproxen, methyl salicylate, diflunisal, indomethacin, sulindac, diclofenac, ketoprofen, ketorolac, carprofen, fenoprofen, mefenamic acid, piroxicam, meloxicam, methotrexate, celecoxib, valdecoxib, parecoxib, etoricoxib, and nimesulide), corticosteroids (e.g., prednisone, betamethasone, budesonide, cortisone, dexamethasone, hydrocortisone, methylprednisolone, prednisolone, tramcinolone, and fluticasone), rapamycin, rhokinase inhibitors, viral CC-chemokine inhibitor (vCCIs), glucocorticoids, steroids, beta-agonists, anticholinergic agents, methyl xanthines, sulphasalazine, dapsone, psoralens, proteins, peptides, DMARDs, glucocorticoids (e.g., dexamethasone), methotrexate, sulfasalazine, chloriquine, gold, gold salt, copper, copper salt, penicillamine, D-penicillamine, cyclosporine, lipoxins, resolvins, cox-2 inhibitors, MAP kinase inhibitors, caspase-1 inhibitors, JNK inhibitors, ERK inhibitors, Syk inhibitor, JAK inhibitors, and protectins. Anti-inflammatory therapeutic agents are also provided in The Pharmacological Basis of Therapeutics, 10th ed., Gilman et al., eds., McGraw-Hill Press (2001) and Remington's Pharmaceutical Science's, 18th ed. Easton: Mack Publishing Co. (1990). In a particular embodiment, the anti-inflammatory therapeutic agent is selected from the group consisting of glucocorticoids, resolvins, cox-2 inhibitors, MAP kinase inhibitors, caspase-1 inhibitors, JNK inhibitors, ERK inhibitors, Syk inhibitor, and JAK inhibitors. In a particular embodiment, the anti-inflammatory therapeutic agent is dexamethasone.

A "bone related therapeutic agent" refers to an agent suitable for administration to a patient that induces a desired biological or pharmacological effect such as, without limitation, 1) increasing bone growth, 2) preventing an undesired biological effect such as an infection, 3) alleviating a condition (e.g., pain or inflammation) caused by a disease associated with bone, and/or 4) alleviating, reducing, or eliminating a disease from bone. In a particular embodiment, the bone related therapeutic agent possesses a bone anabolic effect and/or bone stabilizing effect. Bone related therapeutic agents include, without limitation, cathepsin K inhibitor, metalloproteinase inhibitor, prostaglandin E receptor agonist, prostaglandin E1 or E2 and analogs thereof, parathyroid hormone and fragments thereof, resolvins and analogs thereof, antimicrobials, glucocorticoids (e.g., dexamethasone) and derivatives thereof, and statins (e.g., simvastatin).

As will be recognized by a person of ordinary skill in the art, anti-inflammatory drugs and imaging agents, as used herein, include acceptable salts, esters, or salts of such esters. For example, glucocorticoids include pharmaceutically acceptable salts and esters thereof, therefore, when a drug is described, e.g., dexamethasone, pharmaceutically acceptable salts thereof are also described, such as dexamethasone palmitate.

The therapeutic agent(s), imaging agent(s), and/or targeting moiety may be linked to the HPMA copolymer backbone by way of a spacer. Spacers (linkers) are known in the art and the person of ordinary skill in the art may select a spacer based on length, reactivity, flexibility and the like. For example, a spacer may be an alkyl or alkyne having from one to 50, preferably one to 15 carbons. A spacer of the invention may also be a peptide sequence (for example, selected from all nature amino acids) having from one to 20, preferably one to 10 residues. The linkages (linker domains) of the instant polymers may be non-degradable or degradable under physiological conditions. In one embodiment, a spacer may contain a bond which is cleavable under acidic pH (e.g., pH <6, particularly <5.5). Examples of pH sensitive linkers comprise, without limitation, a hydrazone bond, acetal bond, cis-aconityl spacer, phosphamide bond, silyl ether bond, etc. Spacers may also be cleaved upon a stimulus including, but not limited to, changes in pH, presence of a specific enzyme (protease) activity (for example, cathespins (e.g., cathepsin K), MMPs, etc.), changes in oxygen levels, etc. In a particular embodiment, the linker/spacer is biodegradable and cleavable under physiological conditions. In another embodiment, when an imaging agent is linked to the HPMA copolymer backbone, the linker/spacer is not degradable or cleavable under physiological conditions.

As stated hereinabove, the HPMA copolymers of the instant invention may comprise at least one therapeutic agent, at least one imaging agent, and/or at least one at targeting moiety. For example, a single polymer may comprise more than one therapeutic agent. As another example, a single copolymer may comprise one or more therapeutic agents and one or more imaging agents. Alternatively, more than one HPMA copolymer may be administered to the subject. For example, a copolymer comprising one or more therapeutic agents may be administered with a copolymer comprising one or more imaging agents.

The instant invention also encompasses compositions comprising at least one HPMA copolymer of the instant invention and at least one pharmaceutically acceptable carrier. The composition may further comprise at least one other anti-inflammatory therapeutic agent. Such composition may be administered, in a therapeutically effective amount, to a patient in need thereof for the treatment and/or imaging of an inflammatory disease or disorder. In a particular embodiment, at least one other anti-inflammatory agent is administered separately from the above composition (e.g., sequentially or concurrently).

The compositions of the present invention can be administered by any suitable route, for example, by injection (e.g., for local (direct) or systemic administration (intravenous)), oral, pulmonary, topical, nasal or other modes of administration. The composition may be administered by any suitable means, including parenteral, intramuscular, intravenous, intraarterial, intraperitoneal, subcutaneous, topical, inhalatory, transdermal, intraocular, intrapulmonary, intrarectal, and intranasal administration. In a particular embodiment, the composition is injected directly to the desired site. In general, the pharmaceutically acceptable carrier of the composition is selected from the group of diluents, preservatives, solubilizers, emulsifiers, adjuvants and/or carriers. The compositions can include diluents of various buffer content (e.g., Tris HCl, acetate, phosphate), pH and ionic strength; and additives such as detergents and solubilizing agents (e.g., Tween 80, Polysorbate 80), anti oxidants (e.g., ascorbic acid, sodium metabisulfite), preservatives (e.g., Thimersol, benzyl alcohol) and bulking substances (e.g., lactose, mannitol). The compositions can also be incorporated into particulate preparations of polymeric compounds such as polyesters, polyamino acids, hydrogels, polylactide/glycolide copolymers, ethylenevinylacetate copolymers, polylactic acid, polyglycolic acid, etc., or into liposomes. Such compositions may influence the physical state, stability, rate of in vivo release, and rate of in vivo clearance of components of a pharmaceutical composition of the present invention. See, e.g., Remington's Pharmaceutical Sciences, 18th Ed. (1990, Mack Publishing Co., Easton, PA 18042) pages 1435 1712 which are herein incorporated by reference. The pharmaceutical composition of the present invention can be prepared, for example, in liquid form, or can be in dried powder form (e.g., lyophilized). In vivo delivery may be accomplished by use of a syrup, an elixir, a liquid, a tablet, a pill, a time-release capsule, an aerosol, a transdermal patch, an injection, a drip, an ointment, etc.

In yet another embodiment, the compositions of the present invention can be delivered in a controlled release system, such as using an intravenous infusion, an implantable osmotic pump, a transdermal patch, liposomes, or other modes of administration. In a particular embodiment, a pump may be used (see Langer, supra; Sefton, CRC Crit. Ref. Biomed. Eng. (1987) 14:201; Buchwald et al., Surgery (1980) 88:507; Saudek et al., N. Engl. J. Med. (1989) 321:574). In another embodiment, polymeric materials may be employed (see Medical Applications of Controlled Release, Langer and Wise (eds.), CRC Press: Boca Raton, Florida (1974); Controlled Drug Bioavailability, Drug Product Design and Performance, Smolen and Ball (eds.), Wiley: New York (1984); Ranger and Peppas, J. Macromol. Sci. Rev. Macromol. Chem. (1983) 23:61; see also Levy et al., Science (1985) 228:190; During et al., Ann. Neurol. (1989) 25:351; Howard et al., J. Neurosurg. (1989) 71:105). In yet another embodiment, a controlled release system can be placed in proximity of the target tissues of the animal, thus requiring only a fraction of the systemic dose (see, e.g., Goodson, in Medical Applications of Controlled Release, supra, (1984) vol. 2, pp. 115 138). In particular, a controlled release device can be introduced into an animal in proximity to the site of inappropriate inflammation. Other controlled release systems are discussed in the review by Langer (Science (1990) 249:1527 1533).

The composition of the instant invention may be administered for the treatment of osteolysis, the inflammation associated therewith, and/or implant loosening. The dosage ranges for the administration of the composition of the invention are those large enough to produce the desired effect (e.g., curing, relieving, and/or preventing the inflammatory disorder, the symptom of it, or the predisposition towards it). The dosage should not be so large as to cause adverse side effects, such as unwanted cross-reactions, anaphylactic reactions, and the like. Generally, the dosage will vary with the age, condition, sex and extent of the disease in the patient and can be determined by one of skill in the art. The dosage can be adjusted by the individual physician in the event of any counter indications. An effective amount of a drug is well known in the art and changes due to the age, weight, severity of a subject's condition, the particular compound in use, the strength of the preparation, and the mode of administration. The determination of an effective amount is preferably left to the prudence of a treating physician, but may be determined using methods well known in the art (The Pharmacological Basis of Therapeutics, 10th ed., Gilman et al. eds., McGraw-Hill Press (2001); Remington's Pharmaceutical Science's, 18th ed. Easton: Mack Publishing Co. (1990)). The compositions of the invention may be prepared using methods known in the art, for example, the preparation of a pharmaceutical composition is known in the art (The Pharmacological Basis of Therapeutics, 10th ed., Gilman et al. eds., McGraw-Hill Press (2001); Remington's Pharmaceutical Science's, 18th ed. Easton: Mack Publishing Co. (1990)).

As stated hereinabove, the invention provides a water-soluble polymeric delivery system for delivery of imaging agents, which are useful for non-invasive imaging, diagnosis, prognosis, and evaluation of orthopedic and/or dental implants. The instant invention encompasses methods of detecting an increased risk for implant (e.g., orthopedic and/or dental implants) loosening and/or osteolysis. Orthopedic implants include structures that are implanted into a living body in order to augment or supplant the bone structure of the patient into whom it is implanted. Examples of orthopedic implants include, without limitation, grafts, plates, meshes, replacement devices (e.g., joint, knee, hip, elbow, wrist, etc.), upper femoral devices, plastic surgery implants, upper humeral devices, shoulder devices, passive tendon devices, spinal devices, finger/toe devices, diaphysis devices, hydroxyapatite-coated metal implant, and metal implants (e.g., stainless steel, titanium, and titanium alloy). The imaging agent containing polymer may also be used to monitor the progress of a treatment. In another exemplary embodiment, the invention provides a method of screening anti-inflammatory therapeutic agents or other compounds for the treatment of osteolysis and/or implant loosening. In one embodiment, the anti-inflammatory agent is attached to a water-soluble polymeric delivery system of the invention and administered to a subject, and the effect of the therapeutic agent is monitored, for example, using an imaging agent, thereby identifying effective therapeutic agents. In another embodiment, the anti-inflammatory agent is administered to a patient and an imaging agent attached to a water-soluble polymeric delivery system of the invention is administered so that the effect of the therapeutic agent is monitored. Optionally, an imaging agent may be co-administered for the purpose of monitoring and/or screening the activity of the anti-inflammatory agent. Optionally, a targeting moiety or moieties may be used in the method of screening.

As stated hereinabove, the polymers of the instant invention are used to treat osteolysis, the inflammation associated therewith, and/or implant loosening. The methods comprise the administration of a HPMA copolymer of the instant invention comprising at least one therapeutic agent to a subject (e.g., human). The instant methods may be used to prevent and/or inhibit implant loosening/failure.

### Definitions

As used herein, "diagnose" refers to detecting and identifying a disease or disorder in a subject. The term may also encompass assessing or evaluating the disease status (progression, regression, stabilization, response to treatment, etc.) in a patient known to have the disease or disorder.

As used herein, the term "prognosis" refers to providing information regarding the impact of the presence of a disease or disorder on a subject's future health (e.g., expected morbidity or mortality, the likelihood/risk of osteolysis, the likelihood/risk of implant loosening, etc.). In other words, the term "prognosis" refers to providing a prediction of the probable course and outcome of a disease or disorder or the likelihood of recovery from the disease or disorder.

The term "treat" as used herein refers to any type of treatment that imparts a benefit to a patient afflicted with a disease or disorder, including improvement in the condition of the patient (e.g., in one or more symptoms), delay in the progression of the condition, etc.

The phrase "effective amount" refers to that amount of therapeutic agent that results in an improvement in the patient's condition.

"Pharmaceutically acceptable" indicates approval by a regulatory agency of the Federal or a state government or listed in the U.S. Pharmacopeia or other generally recognized pharmacopeia for use in animals, and more particularly in humans.

A "carrier" refers to, for example, a diluent, adjuvant, preservative (e.g., Thimersol, benzyl alcohol), anti-oxidant (e.g., ascorbic acid, sodium metabisulfite), solubilizer (e.g., Tween 80, Polysorbate 80), emulsifier, buffer (e.g., Tris HCl, acetate, phosphate), bulking substance (e.g., lactose, mannitol), excipient, auxilliary agent or vehicle with which an active agent of the present invention is administered. Pharmaceutically acceptable carriers can be sterile liquids, such as water and oils, including those of petroleum, animal, vegetable or synthetic origin, such as peanut oil, soybean oil, mineral oil, sesame oil and the like. Water or aqueous saline solutions and aqueous dextrose and glycerol solutions are preferably employed as carriers, particularly for injectable solutions. The compositions can be incorporated into particulate preparations of polymeric compounds such as polylactic acid, polyglycolic acid, etc., or into liposomes or micelles. Such compositions may influence the physical state, stability, rate of in vivo release, and rate of in vivo clearance of components of a pharmaceutical composition of the present invention. The pharmaceutical composition of the present invention can be prepared, for example, in liquid form, or can be in dried powder form (e.g., lyophilized). Suitable pharmaceutical carriers are described in "Remington's Pharmaceutical Sciences" by E.W. Martin (Mack Publishing Co., Easton, PA); Gennaro, A. R., Remington: The Science and Practice of Pharmacy, 20th Edition, (Lippincott, Williams and Wilkins), 2000; Liberman, et al., Eds., Pharmaceutical Dosage Forms, Marcel Decker, New York, N.Y., 1980; and Kibbe, et al., Eds., Handbook of Pharmaceutical Excipients (3rd Ed.), American Pharmaceutical Association, Washington, 1999.

The term "isolated" refers to the separation of a compound from other components present during its production. "Isolated" is not meant to exclude artificial or synthetic mixtures with other compounds or materials, or the presence of impurities that do not substantially interfere with the fundamental activity, and that may be present, for example, due to incomplete purification, or the addition of stabilizers.

"Linker", "linker domain", and "linkage" refer to a chemical moiety comprising a covalent bond or a chain of atoms that covalently attaches at least two compounds, for example, a targeting moiety to a therapeutic agent. The linker can be linked to any synthetically feasible position of the compounds, but preferably in such a manner as to avoid blocking the compounds desired activity. Linkers are generally known in the art. Exemplary linkers may comprise at least one optionally substituted; saturated or unsaturated; linear, branched or cyclic alkyl group or an optionally substituted aryl group. In a particular embodiment, the linker may contain from 0 (i.e., a bond) to about 500 atoms, about 1 to about 100 atoms, or about 1 to about 50 atoms. The linker may also be a polypeptide (e.g., from about 1 to about 20 amino acids). The linker may be biodegradable under physiological environments or conditions. The linker may also be may be non-degradable and can be a covalent bond or any other chemical structure which cannot be cleaved under physiological environments or conditions.

As used herein, the term "biodegradable" or "biodegradation" is defined as the conversion of materials into less complex intermediates or end products by solubilization hydrolysis under physiological conditions, or by the action of biologically formed entities which can be enzymes or other products of the organism. The term "non-degradable" refers to a chemical structure that cannot be cleaved under physiological condition, even with any external intervention. The term "degradable" refers to the ability of a chemical structure to be cleaved via physical (such as ultrasonication), chemical (such as pH of less than 6 or more than 8) or biological (enzymatic) means.

As used herein, the term "bone-targeting" refers to the capability of preferentially accumulating in hard tissue rather than any other organ or tissue, after administration in vivo.

The following example is provided to illustrate certain embodiments of the invention. It is not intended to limit the invention in any way.

### EXAMPLE

### Materials and Methods

### Synthesis of Poly (HPMA-co-APMA)

N-(2-hydroxypropyl)methacrylamide (HPMA; 1 g, 6.98 mmol; Kopecek et al. (1973) Eur. Polymer J., 9:7-14), N-(3-aminopropyl)methacrylamide hydrochloride (APMA, 12.5 mg, 0.07 mmol, Polysciences, Inc., Warrington, PA), azobisisbutyronitrile (AIBN, 6.74 mg, 41 µmol, Sigma-Aldrich, Milwaukee, WI), S,S'-bis(α,α'-dimethyl-α"-acetic acid)-trithiocarbonate (CTA, 6.26 mg, 23.6 µmol, purity > 97%) (Lai et al. (2002) Macromolecules 35:6754-6) and N-methacryloylaminopropyl fluorescein thiourea (MA-FITC, 4 mg) (Omelyanenko et al. (1998) J. Control Release 53:25-37) were dissolved in 8 mL methanol, placed in an ampoule, and purged with N₂ for 5 minutes. The ampoule was flame-sealed and maintained at 40°C for 48 hours avoiding of light. The product was purified by LH-20 column (GE HealthCare, Piscataway, NJ) to remove the unreacted low molecular weight compounds and then lyophilized. The final yield was 0.42 g. The amine content of the copolymer was determined as 2.7 × 10⁻⁵ mol/g using the ninhydrin assay (Moore et al. (1954) J. Biol. Chem., 211:907-13). The weight average molecular weight (M_{w} = 3.71 × 10⁴) and number average molecular weight (Mₙ = 2.65 × 10⁴) of copolymers were determined based on HPMA homopolymer calibration using an AKTA FPLC system (GE HealthCare, Piscataway, NJ) equipped with UV and RI detectors. See also Liu et al. (Pharm. Res. (2008) 25:2910-2919).

### Synthesis of IRDye 800CW-labeled HPMA copolymer (Figure 1)

Poly (HPMA-co-APMA) (16.5 mg, containing 0.00044 mmol of amine), IRDye 800CW NHS Ester (LI-COR® Biosciences, Lincoln, NE) (0.5 mg, 0.00043 mmol) were dissolved in 300 µL of DMF with 5 µL of N, N-diisopropylethylamine added. The solution was stirred overnight in darkness at room temperature. The product was then purified on a LH-20 column and lyophilized. The final product yield was 15.02 mg. The IRDye 800CW content was determined as 1.1 × 10⁻⁵ mol/g using Lambda 10 UV/Vis Spectrometer (PerkinElmer, Shelton, CT).

### Synthesis of Alexa Fluor® 488-labeled HPMA copolymer (Figure 1)

Poly (HPMA-co-APMA) (30.0 mg, 0.0008 mmol), Alexa Fluor® 488 NHS ester (0.517 mg, 0.0008 mmol, Invitrogen, Camarillo, CA) was dissolved in 0.5 mL of DMF with 5 µL of N, N-diisopropylethylamine added. The mixture was stirred overnight in darkness at room temperature. The product was purified on LH-20 column and lyophilized. The final product yield was 28.62 mg. The Alexa Fluor® 488 content was determined as 1.9 × 10⁻⁵ mol/g using Lambda 10 UV/Vis Spectrometer.

### Mouse calvaria osteolysis model

Poly(methyl methacrylate) (PMMA) particles (1-10 µm, Bangs Laboratories, Fishers, IN) were soaked in 70% ethanol overnight, then washed and suspended in sterile phosphate-buffered saline (PBS) prior to implantation. A limulus assay was performed using Pyrosate® kit (Associates of Cape Cod, Inc., East Falmouth, MA) to confirm that the treated particles were endotoxin-free. Male Swiss Webster mice (6 weeks, Charles River Laboratories Inc., Wilmington, MA) were anesthetized with 70-80 mg/kg of ketamine and 5-7 mg/kg xylazine by intraperitoneal injection. A 25G needle was inserted subcutaneously to remove periosteum from calvaria surface by scratching with the needle tip 30 times. PBS (100 µL) or PMMA (30 mg suspended in 100 µL PBS) was then deposited onto the calvarial surface through the inserted needle. All animal experiments were performed according to a protocol approved by University of Nebraska Medical Center Institutional Animal Care and Use Committee.

### Near infrared optical imaging of mice implanted with PMMA particles

One-day post PMMA particle implantation, the mice were given P-IRDye (0.5 mg/mice) via tail vein injection. The group injected with PBS was used as a negative control and was given the same dose of P-IRDye. The mice were imaged prior to and daily after contrast agent injection using an XENOGEN IVIS® 200 Series Imaging System (Hopkinton, MA) to evaluate the distribution of the HPMA copolymer conjugate continuously for the 6 days.

### Micro-CT analyses of mouse calvaria

At necropsy, the calvaria were removed by dissecting bone free from the underlying brain tissue and removing an elliptical plate of bone bound by the foramen magnum, auditory canals and orbits. They were stored in 70% ethanol and then scanned using a Scanco µCT35 (Scanco Medical, Brüttisellen, Switzerland) system with a resolution of 15 µm at regular contrast conditions (55 KVp, 145 µA, 0.36 degrees angular rotation step). Three-dimensional reconstructions of the scanned volumes by the system reconstruction software and bone background segmentation were then performed.

### Histological evaluation

For identification of osteoclasts, whole calvaria were fixed in 70% ethanol solution and then TRAP stained using a commercial staining kit (387A, Sigma-Aldrich, St. Louis, MO). Purple-stained cells are recognized as TRAP positive osteoclasts. For evaluation of bone resorption, the calvaria were fixed in 4% neutralized paraformaldehyde for 24 hours and then decalcified in 10% EDTA (with 0.5% paraformaldehyde in PBS) at 4°C for 2 weeks. The decalcification solution was changed every 2 days. The specimens were then paraffin embedded, sectioned (5 µm thickness) and H&E stained. Both the TRAP stained calvaria and H&E sections were examined with an Olympus BX51 microscopy (Olympus, Japan).

### Immunohistochemical analysis

Six days post particle implantation, P-Alexa (4.0 mg/mice) was given to mice by tail vein injection. At necropsy (24 hours post injection), the upper skull (including skin, underlying soft tissue and calvaria) was isolated as described above. The tissue was cut into two halves in the coronal plane centered over the area of particle deposition, immediately embedded in O.C.T. compound and frozen with dry ice for sectioning (7 µm thickness). The slides obtained were first incubated with 10 % goat, rabbit or donkey serum (Sigma-Aldrich, St. Louis, MO) for 30 minutes at room temperature. After addition of the primary antibodies {rabbit anti-mouse prolyl-4-hydroxylase (P4HB, Abcam, Cambridge, MA), rat anti-mouse F4/80 (Invitrogen, Camarillo, CA), rat anti-mouse Ly-6G (Gr-1, Gr1) (Ebioscience, San Diego, CA) and Armenian hamster anti-mouse CD11c (BD Pharmingen, San Jose, CA), diluted in 10% corresponding blocking serum}, the sections were incubated at room temperature for 1 h in a humidified chamber. After washing with PBS, diluted secondary antibody {phycoerythrin (PE) labeled donkey anti-rabbit IgG (Ebioscience, San Diego, CA), PE labeled goat anti-rat IgG (Invitrogen, Camarillo, CA) or PE labeled rabbit anti-Armenian hamster IgG (Ebioscience, San Diego, CA)} was added and incubated for another 30 minutes at room temperature in the dark. In control experiments, primary antibodies were replaced by corresponding isotype controls {purified rabbit IgG (Sigma-Aldrich, St. Louis, MO), purified rat IgG (Sigma-Aldrich, St. Louis, MO) and purified hamster IgG (Jackson ImmunoResearch, West Grove, PA)} and the samples were processed similarly as described above. The processed tissue sections were then evaluated with a Nikon Swept Field confocal microscope. Photos were taken at a magnification of 400×.

### Fluorescence-activated cell scanning (FACS) analysis

Similar to the immunohistochemistry study, P-Alexa (2.0 mg/mice) was given to mice by tail vein injection at six days post particle implantation. At necropsy (24 hours post injection), soft tissues between skin and calvaria were isolated and minced aseptically. The tissues were further digested with collagenase type I (1 mg/mL, Sigma-Aldrich, St. Louis, MO) at 37°C for 2 hours. After passing through a 70 µm cell strainer, a single cell suspension (1 × 10⁶ cells/mL) was obtained. ACK Lysing Buffer (Quality Biological, Gaithersburg, MD) was then used to remove the red blood cells. For FACS evaluation of CD11c, F4/80 and Ly-6G (Gr-1, Gr1) positive cells, the samples were incubated with antibodies {Allophycocyanin (APC)-labeled hamster anti mouse CD11c (BD Pharmingen, San Jose, CA), PE-labeled rat anti-mouse F4/80 (AbD Serotec, Raleigh, NC), PE-labeled rat anti-mouse Ly-6G (Gr-1, Gr1) (Ebioscience, San Diego, CA)} for 30 minutes on ice. For FACS evaluation of P4HB positive cells, the samples were first incubated with rabbit anti-mouse P4HB for 30 minutes on ice and then incubated with PE-labeled donkey anti-rabbit IgG for another 30 minutes on ice. Isotype-matched APC-labeled hamster IgG1 (BD Pharmingen, San Jose, CA), PE-labeled rat IgG2b (BD Pharmingen, San Jose, CA) and purified rabbit IgG were used as negative controls. After the final wash, the cells were analyzed with Becton Dickinson FACSCalibur™ flow cytometer.

### Preliminary in vivo treatment study

One-day post PMMA particle implantation, the mice were randomly assigned into two groups and treated with P-Dex (Figure 2, equivalent Dex dose = 20 mg/kg) and saline via tail vein injection. At 6 days post treatment, the mice were euthanized and the upper skulls of the animals were isolated with the skin, underlining soft tissues and the brain tissue removed. Tissues were then fixed with 70 % ethanol and subjected to micro-CT analysis as described previously.

### Macrophage cultures

CD14-positive monocytes were prepared from PBMCs derived from deidentified normal human donors as described previously (Rakshit et al. (2006) J. Bone Joint Surgery 88:788-99). Cells were cultured for 24 hours at a cell density of 10⁶ cells/mL in α-MEM medium (Invitrogen, Carlsbad, CA) supplemented with 10% fetal bovine serum (VWR, West Chester, PA) and 1% antibiotic/antimycotic (Invitrogen, Carlsbad, CA) in the presence of 10 ng/mL human M-CSF (Peprotech, Rocky Hill, NJ) in 24-well tissue culture plates (1 mL/well). Cells were then pulsed for 4 hours with P-Dex (5 µM) or free Dex (dexamethasone phosphate sodium, 0.6 µM), washed and replenished with fresh medium. The two treatments had equivalent doses of Dex. After 24 hours, cells were treated +/- PMMA particles (30 particles/cell) for 3 hours. Total cellular RNA was prepared (RNeasy, Qiagen) and analyzed by real-time RT-PCR as previously described for inflammatory cytokines (IL1-α, IL-1β). Conditioned media was also analyzed for inflammatory cytokines (TNF, IL1, IL6) by ELISA.

### Statistical Methods

Comparisons between two groups were evaluated with Student's t-test: two-sample two-tails assuming equal variances. A p value of less than 0.05 is considered as statistically significant.

### Results

### Assessment of bone resorption in the mouse calvaria osteolysis model

Micro-CT analysis of calvarial bone specimens revealed extensive focal regions of bone loss associated with sites of PMMA particle implantation compared to PBS treated animals (Figure 3-B1). Quantitatively, the PBS control group had smaller average bone surface to bone volume (BS/BV) values of 16.64 ± 0.36 mm⁻¹ compared to the PMMA group (19.49 ± 1.60 mm⁻¹) (p < 0.05). The bone thickness in the PMMA group was 0.1027 ± 0.0084 cm, which is significantly smaller than that of PBS group of 0.1200 ± 0.0028 cm (p < 0.05). As shown in Figure 4B, multiple TRAP-positive cells were present on the bone surfaces at the sites of PMMA particle implantation, consistent with active osteoclastic bone resorption. H&E staining of decalcified calvaria at the PMMA deposition sites revealed the presence of an intense inflammatory cell infiltrate (Figure 4D).

### Near-infrared optical imaging study

A strong near-infrared (NIR) signal from P-IRDye was associated with the PMMA particle deposition site 2 days post particle implantation (Figure 5). Daily imaging demonstrated persistence of the P-IRDye signal for over 7 days. Quantitative analysis of the NIR signal intensity confirmed that at 7 days post P-IRDye administration ~60 % of the signal remained, consistent with retention of P-IRDye at the PMMA particle implantation site. The distribution and retention of the P-IRDye was significantly lower in the PBS control group compared to the PMMA treated group (p < 0.05).

### Immunohistochemical analysis of mouse calvaria frozen sections

As shown in Figure 6, immunohistochemical staining with anti-F4/80, anti-CD11c, anti-Ly-6G (Gr-1, Gr1) and anti-P4HB antibodies of the frozen sections from the mouse calvaria tissue from PMMA implanted animals revealed that many P-Alexa-positive cells stained positively for Ly-6G (Gr-1, Gr1) or F4/80. CD11c positive cells were either weakly positive or negative for Alexa staining, whereas P4HB positive cells were not detected.

### FACS analysis of cells isolated from PMMA implantation sites

FACS analysis revealed that more than 80% of the cells isolated from the inflammatory sites were Ly-6G (Gr-1, Gr1) positive and of these cells, -18% were P-Alexa positive cells. As shown in Figure 7, 25.1% of the P-Alexa positive cells were F4/80 positive; 35.15% of the P-Alexa positive cells were Ly-6G (Gr-1, Gr1) positive and 9.73% of the P-Alexa positive cells were CD11c positive. All data presented were isotype-control corrected. The study was repeated with P-Alexa administration on day 1 post PMMA particle implantation and tissue isolation and processing on day 7 post particle implantation. Though overall P-Alexa positive cell numbers were reduced, they were still identified as F4/80, Ly-6G or CD11c positive.

### Effects of P-Dex treatment

The therapeutic effect of P-Dex on osteolysis was evaluated by micro-CT. The saline control group had significantly smaller (p < 0.05) bone volume/tissue volume (BV/TV) values of 0.8387 ± 0.0202 compared to the P-Dex group (0.8618 ± 0.0056). The bone mineral density (BMD) value in control group was 836.4 ± 9.1 mg/cm³, which is significantly lower (p < 0.05) than the P-Dex group (858.4 ± 8.7 mg/cm³).

### The effect of P-Dex on inflammatory cytokine expression by PMMA particle-treated human monocytes

As shown in Figure 8, PMMA particle challenge leads to marked increases in IL-1α and IL-1β mRNA expression in cultured human monocytes. Pretreatment of the cells with P-Dex or Dex resulted in a significant repression of the particle-induced pro-inflammatory cytokine response (e.g., TNF and IL-1).
The following represent possible embodiments according to the invention:
Paragraph 1. A method of detecting implant loosening in a subject, said method comprising administering to the subject a composition comprising at least one water soluble polymer and at least one pharmaceutically acceptable carrier, wherein said water soluble polymer is operably linked to at least one imaging agent,
   wherein the presence of said water soluble polymer at the site of the implant in said subject is indicative of implant loosening.
Paragraph 2. The method of paragraph 1, wherein said water soluble polymer is a N-(2-hydroxypropyl)methacrylamide (HPMA) copolymer.
Paragraph 3. The method of paragraph 1, wherein said imaging agent is a radioisotope.
Paragraph 4. The method of paragraph 1, wherein said subject is a human.
Paragraph 5. The method of paragraph 1, wherein said implant is an orthopedic or dental implant.
Paragraph 6. A method of determining an increased risk for osteolysis in a subject, said method comprising administering to the subject a composition comprising at least one water soluble polymer and at least one pharmaceutically acceptable carrier, wherein said water soluble polymer is operably linked to at least one imaging agent,
   wherein the localization of said water soluble polymer at the site in said subject is indicative of an increased risk for osteolysis.
Paragraph 7. The method of paragraph 6, wherein said water soluble polymer is a N-(2-hydroxypropyl)methacrylamide (HPMA) copolymer.
Paragraph 8. The method of paragraph 6, wherein said imaging agent is a radioisotope.
Paragraph 9. The method of paragraph 6, wherein said subject is a human.
Paragraph 10. The method of paragraph 6, wherein said subject has an orthopedic or dental implant.
Paragraph 11. The method of paragraph 10, wherein said water soluble polymer localizes to peri-implant space.
Paragraph 12. A method of inhibiting the loosening of an implant in a subject, said method comprising administering to the subject a composition comprising at least one water soluble polymer and at least one pharmaceutically acceptable carrier, wherein said water soluble polymer is operably linked to at least one therapeutic agent,
   wherein the presence of said water soluble polymer at the site of the implant in said subject inhibits bone destruction and implant loosening.
Paragraph 13. The method of paragraph 12, wherein said water soluble polymer is a N-(2- hydroxypropyl)methacrylamide (HPMA) copolymer.
Paragraph 14. The method of paragraph 12, wherein said therapeutic agent is an anti-inflammatory therapeutic agent.
Paragraph 15. The method of paragraph 14, wherein said anti-inflammatory therapeutic agent is dexamethasone.
Paragraph 16. The method of paragraph 12, wherein said subject is a human.
Paragraph 17. The method of paragraph 12, wherein said implant is an orthopedic or dental implant.
Paragraph 18. The method of paragraph 12, wherein said water soluble polymer is operably linked to said therapeutic agent via a pH-sensitive linker.
Paragraph 19. The method of paragraph 18, wherein said pH-sensitive linker comprises a hydrazone.
Paragraph 20. The method of paragraph 12, further comprising the administration of at least one additional anti-inflammatory therapeutic agent.
Paragraph 21. A method of inhibiting osteolysis in a subject, said method comprising administering to the subject a composition comprising at least one water soluble polymer and at least one pharmaceutically acceptable carrier, wherein said water soluble polymer is operably linked to at least one therapeutic agent, thereby inhibiting said osteolysis. Paragraph 22. The method of paragraph 21, wherein said water soluble polymer is a N-(2- hydroxypropyl)methacrylamide (HPMA) copolymer.
Paragraph 23. The method of paragraph 20, wherein said anti-inflammatory therapeutic agent is dexamethasone.
Paragraph 24. The method of paragraph 23, wherein said anti-inflammatory therapeutic agent is dexamethasone.
Paragraph 25. The method of paragraph 21, wherein said subject is a human.
Paragraph 26. The method of paragraph 21, wherein said osteolysis is at the site of an orthopedic or dental implant.
Paragraph 27. The method of paragraph 21, wherein said HPMA copolymer is operably linked to said therapeutic agent via a pH-sensitive linker.
Paragraph 28. The method of paragraph 27, wherein said pH-sensitive linker comprises a hydrazone.
Paragraph 29. The method of paragraph 21, further comprising the administration of at least one additional anti-inflammatory therapeutic agent.

While certain of the preferred embodiments of the present invention have been described and specifically exemplified above, it is not intended that the invention be limited to such embodiments. Various modifications may be made thereto without departing from the scope and spirit of the present invention, as set forth in the following claims.

## Claims

1. A composition for use in inhibiting the loosening of an implant, said composition comprising: at least one water soluble polymer, wherein said water soluble polymer is operably linked to at least one therapeutic agent.

2. A composition for use according to claim 1, said composition comprising:
at least one water soluble polymer and at least one pharmaceutically acceptable carrier, wherein said water soluble polymer is operably linked to at least one therapeutic agent, wherein said water soluble polymer is a N-(2-hydroxypropyl)methacrylamide (HPMA) copolymer, wherein said N-(2-hydroxypropyl)methacrylamide copolymer consists of N-(2-hydroxypropyl)methacrylamide and N-methacryloyl glycylglycyl hydrazone glucocorticoid, or a pharmaceutically acceptable salt thereof; or
a water-soluble polymer and a glucocorticoid or pharmaceutically acceptable salt thereof, wherein said water-soluble polymer consists of N-(2-hydroxypropyl) methacrylamide (HPMA), and wherein the water-soluble polymer is linked to the glucocorticoid or pharmaceutically acceptable salt thereof via a spacer comprising a hydrazone bond.

3. A composition for use according to claim 2, wherein said glucocorticoid is dexamethasone or dexamethasone palmitate.

4. A composition for use according to claim 2, wherein said implant is an orthopedic or dental implant.

5. A composition for use according to claim 2, wherein said water soluble polymer is operably linked to said therapeutic agent via a pH-sensitive linker, wherein said linker comprises a bond selected from the group consisting of a hydrazone bond, an acetal bond, cis-aconityl spacer, phosphamide bond, and a silyl ether bond.

6. A composition for use in inhibiting osteolysis, said composition comprising at least one water soluble polymer, wherein said water soluble polymer is operably linked to at least one therapeutic agent.

7. A composition for use according to claim 6, said composition comprising:
at least one water soluble polymer and at least one pharmaceutically acceptable carrier, wherein said water soluble polymer is operably linked to at least one therapeutic agent, wherein said water soluble polymer is a N-(2-hydroxypropyl)methacrylamide (HPMA) copolymer, wherein said N-(2-hydroxypropyl)methacrylamide copolymer consists of N-(2-hydroxypropyl)methacrylamide and N-methacryloyl glycylglycyl hydrazone glucocorticoid, or a pharmaceutically acceptable salt thereof; or
a water-soluble polymer and a glucocorticoid or pharmaceutically acceptable salt thereof, wherein said water-soluble polymer consists of N-(2-hydroxypropyl) methacrylamide (HPMA), and wherein the water-soluble polymer is linked to the glucocorticoid or pharmaceutically acceptable salt thereof via a spacer comprising a hydrazone bond.

8. A composition for use according to claim 7, wherein said glucocorticoid is dexamethasone or dexamethasone palmitate.

9. A composition for use according to claim 7, wherein said implant is an orthopedic or dental implant.
